# EUROPÄISCHE PATENTSCHRIFT

(11) **EP 1 561 440 B1**
(45) Veröffentlichungstag und Bekanntmachung des Hinweises auf die Patenterteilung: **08.04.2009**
(21) Anmeldenummer: 04002326.9
(22) Anmeldetag: 03.02.2004
(51) Int. Cl.: A61F 9/008

(54) **Ophtalmologische Vorrichtung**
Ophtalmological device
Outil ophtalmologique

(43) Veröffentlichungstag der Anmeldung: 10.08.2005
(73) Patentinhaber: Iroc AG, 8008 Zürich (CH)
(72) Erfinder: Mrochen, Dr. Michael, 8606 Nänikon (CH); Seiler, Prof. Dr. Theo, 8006 Zürich (CH)
(74) Vertreter: von Hellfeld, Axel

(56) Entgegenhaltungen:
- WO-A-99/45869
- US-A- 3 760 807
- US-A1- 2001 016 731
- WOLLENSAK, SPÖRL ET AL: "Corneal endothelial cytotoxicity of riboflavin/uva treatment in vitro" OPHTALMIC RESEARCH, Nr. 35, 30. Juni 2003 (2003-06-30), Seiten 324-328, XP002285679

## Beschreibung

Die Erfindung betrifft eine Vorrichtung zum Korrigieren einer Fehlsichtigkeit oder Hornhauterkrankung eines Auges sowie Einrichtungen zur Verwendung in einer solchen Vorrichtung.

Der sogenannte Keratokonus ist eine Erkrankung, bei der es zur Erweichung der Augenhornhaut kommt und infolge dieser Erweichung zu einer Auswölbung der Hornhaut aufgrund des Augeninnendruckes. Es versteht sich, dass eine solche Auswölbung zu einer Störung der Abbildungseigenschaften des Auges führt. Eine konservative Therapie des Keratokonus sieht eine Verfestigung der Hornhaut vor. Dies wird zum Beispiel in folgenden Veröffentlichungen beschrieben: E. Spörl, J. Schreiber, K. Hellmund, T. Seiler und P. Knuschke in DER OPHTALMOLOGE 3 - 2000, S. 203-206; E. Spörl, T. Seiler in JOURNAL OF REFRACTIVE SURGERY, Vol. 15, 1999, S. 711-713; G. Wollensak, E. Spoerl, T. Seiler, AMERICAN JOURNAL OF OPHTALMOLOGY, May 2003, S. 620-627. Kurz gesagt, wird gemäß diesem Stand der Technik zur konservativen Therapie des Keratokonus zunächst das Epithel von der Hornhaut entfernt und dann ein Fotosensibilisator (z.B. Riboflavin) auf die freigelegte Hornhaut getropft. Dieser Fotosensibilisator durchdringt dann die gesamte Hornhaut und gelangt auch in die Vorderkammer des Auges. Sodann wird das Auge mit ausgewählter elektromagnetischer Strahlung bestrahlt (zum Beispiel UVA oder UV) und dadurch werden biochemische und biomechanische Prozesse induziert (zum Beispiel ein "Cross-linking" bzw. eine Vernetzung), welche zu einer Verfestigung der Hornhaut führen. Der Fotosensibilisator wird als körpereigenes Produkt rückstandslos danach innerhalb relativ kurzer Zeit abgebaut. Die erreichte mechanische Verfestigung des Gewebes verhindert mehr oder weniger die genannte unerwünschte Auswölbung der Hornhaut.

Eine andere bekannte Korrektur einer Fehlsichtigkeit des Auges ist die sogenannte Orthokeratologie. Bei dieser konservativen Therapie wird dem Patienten (zum Beispiel über Nacht) eine spezielle Kontaktlinse aufgesetzt, welche die Hornhaut in gewünschter Weise verformt. Bleibt die verformende Kontaktlinse über einen längeren Zeitraum, zum Beispiel mehrere Stunden auf dem Auge, dann kann die verformende Wirkung nach Abnahme der Kontaktlinse über mehr oder weniger lange Zeitspannen andauern und so eine Verminderung der Fehlsichtigkeit bewirken. Allerdings ist dieser Korrektureffekt nicht stabil, insbesondere bei Patienten mit schwachen mechanischen Eigenschaften der Hornhaut. Auch können Patienten die bei diesem Verfahren auftretende Schwankung der Refraktionseigenschaften des Auges als störend empfinden.

Die US 2001/016731 A1 beschreibt eine Vorrichtung zum Verformen der Hornhaut mit einer Einrichtung zum Verfestigen der Hornhaut unter Verwendung von Strahlung.

Mit der vorliegenden Erfindung sollen eine Vorrichtung und ein Verfahren bereitgestellt werden, mit denen die vorstehend erläuterten Abbildungsfehler und Schwächen des Auges wirksamer behandelt werden können.

Die erfindungsgemäße Vorrichtung ist im Patentanspruch 1 beschrieben und eine Einrichtung zum Einsatz in einer solchen Vorrichtung in Anspruch 3. Weiterhin betrifft die Erfindung auch ein Operationsmikroskop, verbunden mit der genannten Einrichtung, und eine Vorrichtung mit einem chirurgischen Lasersystem für refraktive Korrekturen der Hornhaut in Verbindung mit der genannten erfindungsgemäßen Vorrichtung.

Dabei kann die Verformung und die Verfestigung der Hornhaut gleichzeitig oder mit zeitlichem Versatz oder zeitlicher Überlappung erfolgen. In der Regel wird die Verfestigung durchgeführt, wenn die Verformung vorliegt.

Die Einrichtung zum Verformen der Hornhaut weist bevorzugt einen Formkörper auf, der auf dem Auge platzierbar ist, also zum Beispiel eine als solches bekannte Kontaktlinse oder dergleichen. Für die erfindungsgemäße Vorrichtung muss jedoch der Formkörper nicht notwendigerweise wie eine das Sehvermögen des Auges optimal verbessernde Kontaktlinse gestaltet sein, vielmehr kann der Formkörper unter Berücksichtigung der nachfolgend näher beschriebenen Verfestigung der Hornhaut optimiert werden.

Die vorstehend erläuterte Einrichtung zum Verformen der Hornhaut weist bevorzugt einen Formkörper auf, der geeignet ist, so auf die Hornhaut aufgebracht zu werden, dass zwischen der Hornhaut und dem Formkörper ein Unterdruck (Vakuum) entsteht, durch welchen die Hornhaut geformt wird, also sich im gesamten gewünschten Bereich satt an die Fläche des Formkörpers anlegt.

Die Verfestigung der in eine gewünschte Form gebrachten Hornhaut erfolgt mit einer erfindungsgemäßen Vorrichtung mit zumindest einer Strahlungsquelle zum Bestrahlen der Hornhaut, wobei die Strahlung bevorzugt homogen auf die zu verfestigende Hornhaut fällt. Eine homogene Verteilung der elektromagnetischen Strahlung liegt dann vor, wenn pro Flächeneinheit im wesentlichen die gleiche Strahlungsmenge auf die Hornhaut auftrifft. Eine solche homogene Strahlungsverteilung wird in der Regel mit einer stationären punktförmigen Strahlungsquelle, deren Strahlung auf die sphärisch gekrümmte Hornhaut trifft, nicht erreicht weil der Einfallswinkel der Strahlung in Abhängigkeit vom Ort auf der Hornhaut variiert. Die Erfindung sieht deshalb besondere Maßnahmen zur Homogenisierung der Strahlungsverteilung vor, sodass auch die mit der Strahlung bewirkte Verfestigung der Hornhaut im wesentlichen homogen ist.

In Abwandlung der vorstehend beschriebenen Ausführungsform der Erfindung ist es auch möglich, Steuereinrichtungen für die Strahlungsverteilung über die Hornhaut so vorzusehen, dass die pro Flächeneinheit auf die Hornhaut auftreffende Strahlungsmenge in Abhängigkeit vom Ort auf der Hornhaut wahlweise einstellbar ist, also beispielsweise so, dass in mehr peripheren Bereichen der Hornhaut eine stärkere Verfestigung erfolgt als in mehr zentralen Bereichen der Hornhaut, oder umgekehrt, je nach Diagnose und/oder Therapieziel.

So sieht eine besondere Ausgestaltung der Erfindung vor, dass eine Einrichtung vorgesehen ist zum Bestimmen von Eigenschaften der Hornhaut und/oder anderer Komponenten des Auges. Diese Messungen können gegebenenfalls an unterschiedlichen Orten der Hornhaut zu unterschiedlichen Ergebnissen führen, was wiederum für die oben erwähnte Steuerung der Verteilung der Intensität der elektromagnetischen Strahlung in Abhängigkeit vom Ort des Auges bei besonderen Ausführungsformen der Erfindung von Bedeutung sein kann.
Die erfindungsgemäße Einrichtung zum Verfestigen der Hornhaut mittels elektromagnetischer Strahlung kann so gestaltet sein, dass sie mit ihrem die Hornhaut formenden Formkörper in Eingriff mit der Hornhaut steht. In Abwandlung dieser Ausführungsform kann aber auch die Einrichtung, mit der die elektromagnetische Strahlung auf die Hornhaut aufgebracht wird, so gestaltet sein, dass sie Abstand von der Hornhaut hat. Die Erfindung lehrt auch unterschiedliche Strahlungsquellen für die elektromagnetische Strahlung und unterschiedliche Techniken, die Strahlung zum Einsatzort zu führen. Einzelheiten dazu finden sich in den abhängigen Patentansprüchen und in der nachfolgenden Beschreibung von Ausführungsbeispielen.

Gemäß einer bevorzugten Ausgestaltung der Erfindung ist vorgesehen, die Einrichtung, mit der die elektromagnetische Strahlung auf die Hornhaut abgestrahlt wird, mit einem Operationsmikroskop zu koppeln, und zwar bevorzugt derart, dass der Operateur während des Aufbringens der elektromagnetischen Strahlung das Auge und insbesondere die Hornhaut, oder Teile davon, beobachten kann.

Gemäß einer anderen bevorzugten Ausgestaltung der Erfindung wird ein als solches bekannter sogenannter "Justierstrahl" für die Positionierung des Auges eingesetzt. Ein solcher Strahl wird in der Literatur auch bisweilen als "Fixierlichtstrahl" bezeichnet. Damit lässt sich die Positionierung des Auges in Bezug auf die beschriebenen Vorrichtungen und Einrichtungen verbessern. Es ist auch möglich, die hier beschriebenen Vorrichtungen und Einrichtungen mit einem sogenannten "eye-tracker" zu kombinieren. Solche "eye-tracker" sind Augenverfolgungssysteme, die mögliche Bewegungen des Auges optisch verfolgen und andere für die Chirurgie eingesetzte Instrumente, zum Beispiel Laserstrahlen, entsprechend der Augenbewegung nachführen. Es ist, gemäß einer anderen Variante der Erfindung, auch möglich, die Positionierung der beschriebenen Vorrichtungen und Einrichtungen am Auge mit einem Brillengestell zu fördern.

Die Erfindung lehrt auch ein Verfahren zum Korrigieren einer Fehlsichtigkeit des Auges, bei dem eine Verformung und eine Verfestigung der Augenhornhaut in Kombination durchgeführt werden.

Weitere bevorzugte Ausgestaltungen der Erfindung ergeben sich aus den abhängigen Patentansprüchen und der nachfolgenden Beschreibung von Ausführungsbeispielen anhand der Zeichnungen. Es zeigt:
- Figur 1: eine Vorrichtung zum Korrigieren einer Fehlsichtigkeit eines Auges;
- Figur 2: eine abgewandelte Ausführungsform einer Vorrichtung zum Korrigieren einer Fehlsichtigkeit des Auges;
- Figur 3: ein weiteres Ausführungsbeispiel einer Vorrichtung zum Korrigieren einer Fehlsichtigkeit des Auges in Kombination mit einem Mikroskop; und
- Figur 4: schematisch eine Anordnung mehrerer Strahlungsquellen zum Bestrahlen einer Hornhaut.

In den Figuren sind einander entsprechende oder funktionsähnliche Bauteile mit gleichen Bezugszeichen versehen.

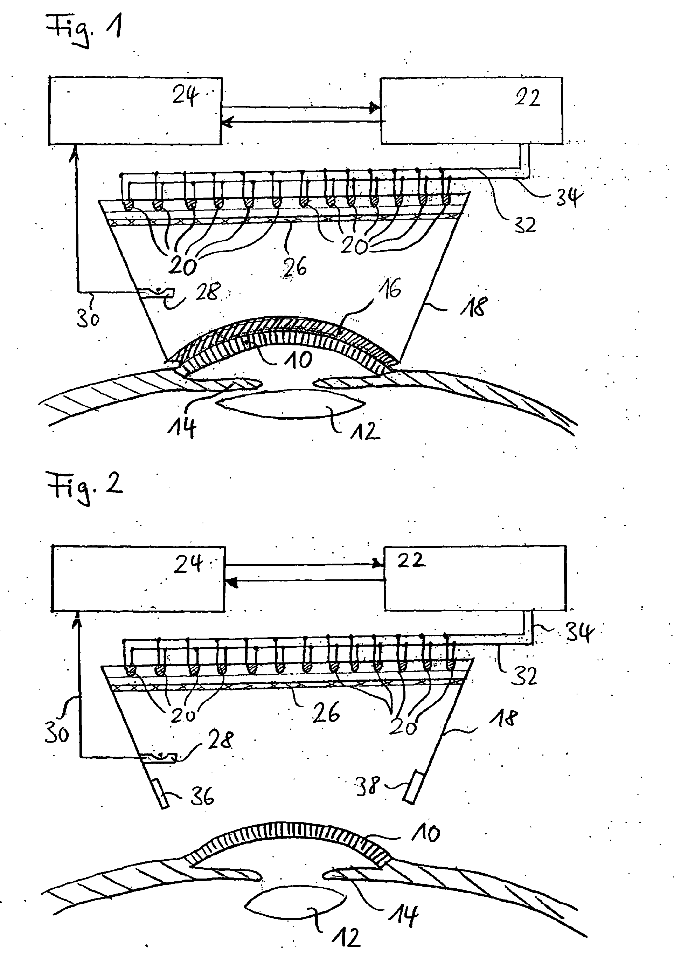

Figur 1 zeigt schematisch ein Auge mit einer Hornhaut 10, einer Linse 12 und einer Iris 14.

Beim Ausführungsbeispiel nach Figur 1 liegt ein Formkörper 16 direkt auf der Hornhaut 10 um diese in gewünschter Weise zu verformen. Ohne den Formkörper 16 (d.h. vor dessen Aufdrücken auf die Hornhaut) hatte die Hornhaut 10 eine andere Form. Der Formkörper 16 ist fest verbunden mit einem Gehäuse 18, das zur Führung elektromagnetischer Strahlung in Richtung auf den Formkörper 16 und die Kornea 10 beim dargestellten Ausführungsbeispiel konisch geformt ist. Zur Strahlungsführung kann das Gehäuse 18 innenseitig verspiegelt sein.

Eine Vielzahl von Strahlungsquellen 20 sind mit dem Gehäuse 18 verbunden. Beim dargestellten Ausführungsbeispiel sind die Strahlungsquellen 20 als LEDs ausgebildet. Mittels einer Stromversorgung 22 werden die einzelnen Strahlungsquellen 20 einzeln einstellbar angesteuert, d.h. die von den einzelnen Strahlungsquellen 20 abgegebene Strahlungsmenge kann selektiv, je nach Bedarf, eingestellt werden. Dabei kann sowohl die von allen Strahlungsquellen 20 abgegebene Strahlungsmenge gleichzeitig proportional eingestellt werden oder es können einzelne Strahlungsquellen, je nach ihrem Ort, wahlweise selektiv hinsichtlich der von ihnen abgegebenen Strahlungsmenge eingestellt werden.

Für die Steuerung der von den Strahlungsquellen 20 jeweils abgegebenen Strahlungsmengen ist eine Steuer- und Regeleinheit 24 vorgesehen, die zum Beispiel rechnergesteuert sein kann.

Im Strahlengang der von den Strahlungsquellen 20 abgegebene Strahlung ist ein sogenannter "Diffusor" 26 angeordnet, zum Beispiel in Form einer Streuplatte (Milchglas), einer Platte mit rauer Oberfläche, oder einem transparenten Körper mit Streuzentren. Die Funktion des Diffusors ist es, die von den Strahlungsquellen 20 abgegebene Strahlung möglichst gleichmäßig zu verteilen, sodass Intensitätsspitzen vermieden werden.

Ein Strahlungssensor 28 erfasst einen Teil der vom Diffusor 26 in Richtung auf den Formkörper 16 bzw. die Kornea 10 gerichteten Strahlung, wobei dieser Teil repräsentativ ist für auf die Kornea 10 auftreffenden Strahlung. Das Messsignal des Sensors 28 wird über eine Leitung 30 in die Steuer- und Regeleinheit 24 zur Verarbeitung übertragen, damit die Steuer- und Regeleinheit 24 entsprechend die Stromversorgungseinheit 22 für die einzelnen Strahlungsquellen 20 ansteuern kann. In Figur 1 sind schematisch Leitungen 32, 34 für die einzelnen Strahlungsquellen 20 dargestellt, jedoch ist bevorzugt vorgesehen, dass jede einzelne Strahlungsquelle 20 selektiv ansteuerbar ist, sodass für die einzelnen Strahlungsquellen unterschiedliche Strahlungsintensitäten vorgesehen werden können.

Beim Ausführungsbeispiel nach Figur 2 ist die Vorrichtung gegenüber dem Ausführungsbeispiel nach Figur 1 dahingehend abgewandelt, dass die Einrichtungen zum Erzeugen und Führen der Strahlung in Richtung auf die Kornea von letzterer beabstandet sind. Das Gehäuse 18 weist hierzu an seinen dem Auge zugekehrten Enden Abstandssensoren 36, 38 auf. Die Vorrichtung gemäß Figur 2 und auch alle anderen hier beschriebenen Vorrichtungen zum Erzeugen und Führen elektromagnetischer Strahlung sind alternativ auch ohne Verwendung eines Formkörpers für die Formung der Kornea verwendbar. Beim Ausführungsbeispiel nach Figur 2 kann ein Formkörper (nicht gezeigt), zum Beispiel eine Kontaktlinse oder dergleichen, direkt auf die Kornea 10 appliziert werden.

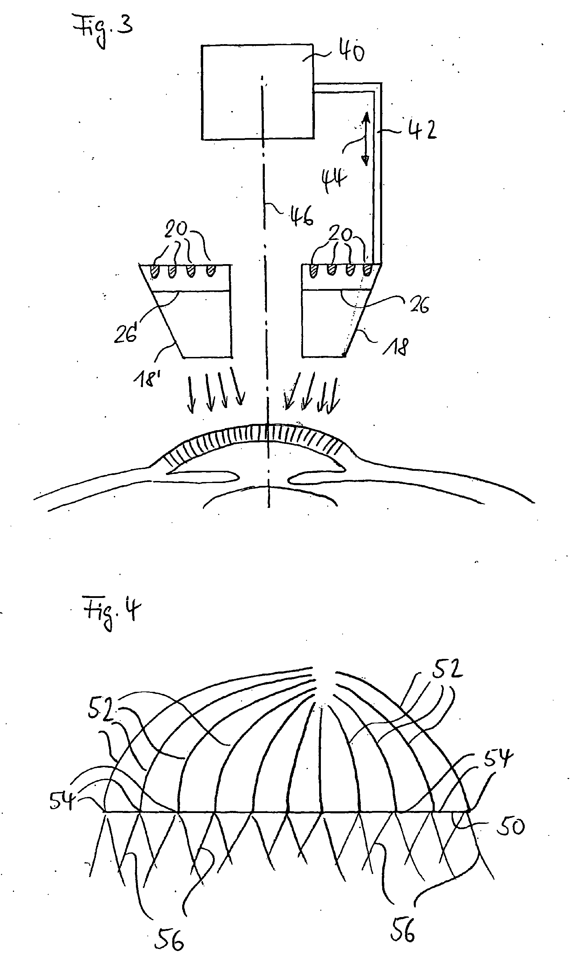

Das Ausführungsbeispiel nach Figur 3 zeigt die Kombination einer abgewandelten Einrichtung zum Erzeugen und Führen elektromagnetischer Strahlung in Verbindung mit einem Mikroskop 40, zum Beispiel einem Operationsmikroskop für die Augenchirurgie. Das Mikroskop 40 kann mit einem Filter versehen sein (nicht gezeigt), das ermöglicht, dass der Operateur ohne Beeinträchtigung durch die von den Strahlungsquellen 20 erzeugte elektromagnetische Strahlung die interessierenden Augenteile beobachten kann. Das Mikroskop 40 ist über einen Arm 42 mit dem Gehäuse 18 der Strahlungsquellen 20 verbunden und zum Beispiel über einen Mechanismus (nicht gezeigt) in Richtung des Doppelpfeiles 44 entlang der optischen Achse 46 bewegbar. Wie dargestellt, weist das Gehäuse 18 mit den Strahlungsquellen 20 zentral im Bereich der optischen Achse 46 einen freien Durchgang für die Mikrokop-Beobachtung auf. Diese Öffnung bildet eine optische Apertur, deren zentrale Achse mit der optischen Achse des Mikroskops übereinstimmt.

Figur 4 zeigt schematisch eine Abwandlung der Vorrichtung zum Erzeugen und Führen der elektromagnetischen Strahlung in Richtung auf die Kornea. Gemäß Figur 4 sind eine Vielzahl von optischen Lichtleitern 52 vorgesehen, deren Enden 54 in einer Halteplatte 50 befestigt sind, sodass die von den Enden abgegebenen Strahlungskegel 56 unterhalb der Platte 50 austreten. Mit einer solchen Anordnung kann in den Figuren 1, 2 und 3 zum Beispiel die Anordnung aus den Strahlungsquellen 20 und dem Diffusor 26 ersetzt werden. Der Abstand zwischen den einzelnen Enden 54 der Lichtleiter 52 und der Abstand der Platte 50 von der Hornhaut können so eingestellt werden, dass sich die Strahlungskegel 56 so weit überlappen, dass eine hinreichend homogene Strahlungsverteilung auf der Hornhaut gegeben ist. Auch bei diesem Ausführungsbeispiel können Halbleiter als Lichtquelle (nicht gezeigt) verwendet werden. Zum Beispiel kann eine gemeinsame Strahlungsquelle (nicht gezeigt) vorgesehen sein, um alle Lichtleiter 52 zu speisen. Es ist auch möglich, einzelne Lichtleiter einzeln anzusteuern, um eine unabhängige Einstellbarkeit der Strahlungsintensität bei zumindest einigen der Lichtleiter zu ermöglichen. Soll mit einer Anordnung gemäß den Figuren 1, 2, 3 oder 4 eine homogene Beaufschlagung der Hornhaut mit elektromagnetischer Strahlung erreicht werden, so ist die sphärische Krümmung der Hornhaut zu berücksichtigen. Diese sphärische Krümmung hat zur Folge, dass die Strahlungen mit unterschiedlichen Winkeln auf die Kornea auftreffen, je nach Abstand von der optischen Achse. Bei einer Anordnung gemäß den Figuren 1 bis 3 wären deshalb zur Erzeugung einer vollständig homogenen Strahlungsverteilung unterschiedliche Ansteuerungen der einzelnen Lichtquellen 20 erforderlich.

Eine einfache Homogenisierung der Strahlungsverteilung kann mit einer Anordnung gemäß Figur 4 dadurch erreicht werden, dass die Platte 50 im gleichen Sinn sphärisch gekrümmt wird, wie die Oberfläche der Kornea. Dann strahlen alle Kegel 56 im wesentlichen radial in Bezug auf ein Zentrum der Sphäre der Kornea, d.h. die Achsen der einzelnen Kegel stehen im wesentlichen senkrecht auf der Oberfläche der Kornea, sodass alle Strahlungskegel 56 in gleicher Weise und mit gleicher Winkelverteilung auf die Oberfläche treffen und dadurch eine homogene Strahlungsverteilung erreicht wird. Der elektronische Steuerungsaufwand hinsichtlich der Strahlungsquellen ist bei dieser Variante wesentlich vereinfacht im Vergleich zu der oben erläuterten Variante, bei der die einzelnen Strahlungsquellen so angesteuert werden, dass sie mit unterschiedlicher Intensität abstrahlen, je nach ihrer Lage in Bezug auf die Kornea.

Mit den anhand der Figuren 1 bis 4 erläuterten Ausführungsbeispielen der Erfindung ist es möglich, die Hornhaut 10 zu formen und zu festigen. Hierfür wird der oben angesprochene Fotosensibilisator in die Hornhaut in der beschriebenen Weise homogen eingebracht und die Bestrahlung mit geeigneten Wellenlängen, zum Beispiel UVA oder UV, durchgeführt. Zur Zeit kommen insbesondere Wellenlängen im UV-Bereich oder härtere Strahlung in Betracht, also Wellenlängen etwa in einem Bereich von 300 bis 400 nm. Die Strahlungsquellen 20 sind entsprechend ausgelegt. Der Formkörper 16 oder eine an dessen Stelle verwendete Kontaktlinse sind für die verwendete Strahlung transparent. Insgesamt kommt grundsätzlich das gesamte elektromagnetische Strahlungsspektrum in Betracht, je nach verwendetem und zur Verfügung stehendem Fotosensibilisator. Es ist auch möglich, eine Hornhaut-Verfestigung ohne Fotosensibilisator nur durch die Strahlung selbst durchzuführen.

Es können für die Strahlungsquellen 20 Leuchtdioden mit unterschiedlichen Wellenlängen eingesetzt werden, je nach den gewünschten therapeutischen Wirkungen. Es ist auch möglich, zusätzlich eine Lichtquelle, deren Strahlung über einen optomechanischen Strahlengang (zum Beispiel einen sogenannten Köhler'schen Strahlengang) geführt wird, zur Beleuchtung einzusetzen.

Gemäß einer bevorzugten Ausgestaltung wird ein Formkörper 16 verwendet, der eine Überverformung der Kornea bewirkt. Es wird also während des Kontaktes zwischen dem Formkörper bzw. der Kontaktlinse mit der Hornhaut letztere stärker verformt als das eigentliche Verformungsziel ist. Damit wird berücksichtigt, dass nach Entfernung des Formkörpers bzw. der Kontaktlinse eine gewisse Regression, d.h. eine Rückbildung der Hornhaut in Richtung auf ihre Ausgangsform erfolgt. Durch die Überverformung wird dann letztlich die gewünschte Form der Hornhaut erreicht. Die Verfestigung mit elektromagnetischer Strahlung kann zumindest teilweise auch bereits vor der Verformung durchgeführt werden; oder auch während und nach der Verformung. Die Verwendung von Befeuchtern, Anästhetika etc. erfolgt je nach Diagnose und Situation.

Die Formung und Verfestigung der Hornhaut mit Vorrichtungen gemäß den Figuren 1 bis 4 kann durch den Einsatz von besonderen Messungen am Auge verbessert werden.

So ist es zum Beispiel mit Mitteln, die der Stand der Technik bereithält, möglich, die Hornhautdicke optisch oder akustisch zu bestimmen. In Abhängigkeit von der so ermittelten Hornhautdicke oder anderen Parameter können dann die Prozessparameter hinsichtlich der Verformung und/oder Verfestigung der Hornhaut, wie oben beschrieben, eingestellt werden. Zum Beispiel wird eine stärkere Hornhaut für eine regressionsfreie Verformung entweder längere Zeiten für die Verfestigung erfordern oder auch eine höhere Konzentration an Fotosensibilisator und/oder eine stärkere Überverformung im oben angesprochenen Sinn.

Eine andere Möglichkeit, die Verformung und Verfestigung mit den Einrichtungen gemäß den Figuren 1 bis 4 zu verbessern ist eine direkte akustische Spektroskopie zur Bestimmung der biomechanischen Eigenschaften der Hornhaut während des Prozesses. Durch Aufbringung von Ultraschall (nicht gezeigt) auf die Hornhaut und Messung der akustischen Transmission können die genannten Eigenschaften der Hornhaut ermittelt werden, zum Beispiel der Grad ihrer Verfestigung während der oben beschriebenen Verfahren. Daraus können insbesondere Steuerparameter für die Zeitspanne der Aufbringung der elektromagnetischen Strahlung und/oder von deren Intensität abgeleitet werden.

Der Stand der Technik kennt auch eine sogenannte dynamische mechanische Spektroskopie zur Bestimmung biomechanischer Eigenschaften der Hornhaut. Auch diese Technik kann in Verbindung mit den gezeigten Vorrichtungen und Verfahren eingesetzt werden, um die Prozessparameter zu optimieren.

Ebenfalls bekannt ist als solches die sogenannte Fluoreszenzanalyse, die besonders geeignet ist, den Bestrahlungsvorgang und die Intensität der aufgebrachten Strahlung sowie deren Wirkungen zu überwachen und aus den ermittelten Werten wiederum Steuerparameter für die Bestrahlung abzuleiten, also zum Beispiel in bestimmten Situationen die Strahlung abzuschwächen, um unerwünschte Effekte zu vermeiden.

Es ist auch möglich, zusammen mit den gezeigten Vorrichtungen die als solches bekannte Konvokalemikroskopie zur Beurteilung von eventuell auftretenden Gewebeeffekten einzusetzen, um unerwünschte Störungen zu vermeiden. Auch kann während des Einsatzes der Vorrichtung vorgesehen sein, den Augeninnendruck zu bestimmen, um daraus eventuell Steuergrößen für die Verfestigung der Hornhaut abzuleiten. Entsprechendes gilt für den Einsatz von als solches bekannten Verfahren für die optische Spektroskopie zur Gewebecharakterisierung oder auch Verfahren, die mittels akustooptischer Spektroskopie eine Gewebecharakterisierung ermöglichen.

Die Stromversorgung der vorstehend beschriebenen Vorrichtungen und Einrichtungen kann wahlweise durch eine Batterie, einen Akkumulator oder durch ein Netzteil erfolgen. Es ist auch möglich, zur Positionierung des Auges des Patienten eine elektromechanisch verfahrbare Patientenliege oder einen entsprechenden Stuhl einzusetzen.

Die vorstehend beschriebenen Vorrichtungen und Einrichtungen können mit einem chirurgischen Lasersystem für refraktive Korrekturen am Auge kombiniert werden. Zum Beispiel kann es sich dabei um ein LASIK-System handeln, das als solches dem Fachmann gut bekannt ist. Mit einer solchen Kombination der erfindungsgemäßen Vorrichtungen mit einem bekannten LASIK-System ist es möglich, zum Beispiel bei einer LASIK-Operation, bei der die Kornea neu geformt wird, eine Vernetzung der Hornhaut durchzuführen, zum Beispiel nach oder während der LASIK-Operation. Damit ist es möglich, den Korrekturbereich bei dem LASIK-Verfahren zu erweitern.

## Patentansprüche

1. Vorrichtung zum Korrigieren einer Fehlsichtigkeit oder Hornhauterkrankung eines Auges, aufweisend die Kombination
- einer Einrichtung (16) zum Verformen der Hornhaut des Auges mit
- einer Einrichtung (18, 20) zum Verfestigen der Hornhaut,
wobei eine oder mehrere Strahlungsquellen (20) in der Einrichtung (18,20) zum Verfestigen der Hornhaut angeordnet sind, **dadurch gekennzeichnet, dass** die eine oder mehreren Strahlungsquellen so angeordnet sind, dass die von ihnen abgegebene Strahlung homogen auf die Hornhaut fällt.

2. Vorrichtung nach Anspruch 1, **dadurch gekennzeichnet, dass** die Einrichtung (16) zum Verformen der Hornhaut einen Formkörper aufweist, der auf dem Auge platzierbar ist.

3. Einrichtung (18, 20) zum Verfestigen einer Hornhaut eines Auges zum Einsatz mit einer Einrichtung (16) zum Verformen der Hornhaut, wobei die Einrichtung zum Verfestigen der Hornhaut eine oder mehrere Strahlungsquellen (20) aufweist, **dadurch gekennzeichnet, dass** die eine oder mehreren Strahlungsquellen so angeordnet sind, dass die von ihnen abgegebene Strahlung homogen auf die Hornhaut fällt.

4. Einrichtung nach Anspruch 3, **dadurch gekennzeichnet, dass** die Einrichtung so gestaltet ist, dass sie bei bestimmungsgemäßem Gebrauch in Kontakt mit der Hornhaut bringbar ist.

5. Einrichtung nach den Ansprüchen 1 oder 3, **dadurch gekennzeichnet, dass** die Einrichtung so gestaltet ist, dass sie bei bestimmungsgemäßem Gebrauch einen vorgegebenen Abstand von der Hornhaut hat.

6. Einrichtung nach einem der Ansprüche 3 bis 5, **dadurch gekennzeichnet, dass** als Strahlungsquelle Leuchtdioden vorgesehen sind.

7. Einrichtung nach einem der Ansprüche 3 bis 5, **gekennzeichnet durch** eine Strahlungsquelle mit Lichtwellenleiter (52).

8. Einrichtung nach einem der Ansprüche 3 bis 7 mit einem konischen Körper (18) zur Strahlungsführung.

9. Einrichtung nach einem der Ansprüche 3 bis 8 mit einem Strahlungssensor (28) zum Erfassen eines Teils der von der Strahlungsquelle bzw. den Strahlungsquellen abgegebenen Strahlung.

10. Einrichtung nach einem der Ansprüche 3 bis 9, **gekennzeichnet durch** eine Steuer- oder Regeleinrichtung (24), mit der die Strahlung steuer- oder regelbar ist.

11. Einrichtung nach Anspruch 5, **gekennzeichnet durch** eine Einrichtung (36, 38) zum Messen des Abstandes zwischen einem Bauteil der Einrichtung und der Hornhaut.

12. Einrichtung nach einem der Ansprüche 3 bis 11, **dadurch gekennzeichnet, dass** die Einrichtung mehrere Strahlungsquellen (20) aufweist, die so angeordnet sind, dass ihre Strahlungskegel (56) durch Überlappung eine homogene Beleuchtung einer Hornhaut ermöglichen.

13. Einrichtung nach einem der Ansprüche 3 bis 12, mit einer Einrichtung (22) zum Ansteuern einzelner Strahlungsquellen.

14. Einrichtung nach einem der Ansprüche 3 bis 13, mit Mitteln zum Bestimmen von Eigenschaften der Hornhaut.

15. Operationsmikroskop, verbunden mit einer Einrichtung gemäß einem der Ansprüche 3 bis 14.

16. Vorrichtung mit einem chirurgischen Lasersystem für refraktive Korrekturen der Hornhaut, in Verbindung mit einer Vorrichtung gemäß einem der Ansprüche 1 bis 15.

## Claims

1. Apparatus for correcting defective vision or corneal disease of an eye, comprising the combination of
- a device (16) for deforming the cornea of the eye with
- a device (18, 20) for hardening the cornea,
wherein one or more radiation sources (20) are arranged in the device (18, 20) for hardening the cornea, **characterized in that** the one or more radiation sources are arranged so that the radiation emitted by them strikes the cornea homogeneously.

2. Apparatus according to claim 1, **characterized in that** the device (16) for deforming the cornea comprises a shaped body which can be placed on the eye.

3. Device (18, 20) for hardening a cornea of an eye for use in a device (16) for deforming the cornea, wherein the device for hardening the cornea comprises one or more radiation sources (20), **characterized in that** the one or more radiation sources are arranged so that the radiation emitted by them strikes the cornea homogeneously.

4. Device according to claim 3, **characterized in that** the device is configured so that it can be brought in contact with the cornea for proper use.

5. Device according to claims 1 or 3, **characterized in that** the device is configured so that it lies at a predetermined distance from the cornea for proper use.

6. Device according to one of claims 3 to 5, **characterized in that** light-emitting diodes are provided as the radiation source.

7. Device according to one of claims 3 to 5, **characterized by** a radiation source with optical waveguides (52).

8. Device according to one of claims 3 to 7, having a conical body (18) for guiding the radiation.

9. Device according to one of claims 3 to 8, having a radiation sensor (28) for detecting a part of the radiation emitted by the radiation source or radiation sources.

10. Device according to one of claims 3 to 9, **characterized by** a control or regulating device (24) which can control or regulate the radiation.

11. Device according to claim 5, **characterized by** a device (36, 38) for measuring the distance between a component of the device and the cornea.

12. Device according to one of claims 3 to 11, **characterized in that** the device comprises a plurality of radiation sources (20) which are arranged so that their radiation cones (56) allow homogeneous illumination of a cornea by overlapping.

13. Device according to one of claims 3 to 12, having a device (22) for driving individual radiation sources.

14. Device according to one of claims 3 to 13, having means for determining properties of the cornea.

15. Operation microscope combined with an instrument according to one of claims 3 to 14.

16. Apparatus having a surgical laser system for refractive corrections of the cornea, in combination with an apparatus or a device according to one of claims 1 to 15.

## Revendications

1. Appareil de correction d'une amétropie ou d'une affection de la cornée d'un oeil, présentant la combinaison
- d'un dispositif (16) de déformation de la cornée de l'oeil avec
- un dispositif (18, 20) de consolidation de la cornée,
dans lequel une ou plusieurs sources de rayonnement (20) sont disposées dans le dispositif (18, 20) de consolidation de la cornée,
**caractérisé en ce que** les une ou plusieurs sources de rayonnement sont disposées de manière à ce que le rayonnement qu'elles délivrent tombe de manière homogène sur la cornée.

2. Appareil selon la revendication 1, **caractérisé en ce que** le dispositif de déformation de la cornée présente une forme qui peut être placée sur l'oeil.

3. Dispositif (18, 20) de consolidation de la cornée d'un oeil pour utilisation avec un dispositif (16) de déformation de la cornée, dans lequel le dispositif de consolidation de la cornée comporte une ou plusieurs sources de rayonnement (20),
**caractérisé en ce que** les une ou plusieurs sources de rayonnement sont disposées de manière à ce que le rayonnement qu'elles délivrent tombe de manière homogène sur la cornée.

4. Dispositif selon la revendication 3, **caractérisé en ce que** le dispositif est constitué de manière à pouvoir, en utilisation conforme à sa destination, être amené en contact avec la cornée.

5. Dispositif selon les revendications 1 ou 3, **caractérisé en ce que** le dispositif est constitué de manière à être, en utilisation conforme à sa destination, à une distance prédéterminée de la cornée.

6. Dispositif selon l'une des revendications 3 à 5, **caractérisé en ce que** des diodes électroluminescentes sont prévues comme sources de rayonnement.

7. Dispositif selon l'une des revendications 3 à 5, **caractérisé par** une source de rayonnement avec guide d'ondes optiques (52).

8. Dispositif selon l'une des revendications 3 à 7, avec un corps conique (18) pour le guidage du rayonnement.

9. Dispositif selon l'une des revendications 3 à 8, avec un capteur de rayonnement (28) destiné à la détection d'une partie du rayonnement délivré par la source de rayonnement ou les sources de rayonnement.

10. Dispositif selon l'une des revendications 3 à 9, **caractérisé par** un dispositif (24) de commande ou de régulation, au moyen duquel on peut commander ou réguler le rayonnement.

11. Dispositif selon la revendication 5, **caractérisé par** un dispositif (36, 38) destiné à la mesure de la distance entre un élément du dispositif et la cornée.

12. Dispositif selon l'une des revendications 3 à 11, **caractérisé en ce que** le dispositif comporte plusieurs sources de rayonnement (20), qui sont disposées de manière à ce que leurs cônes de rayonnement (56) permettent par recouvrement une illumination homogène de la cornée.

13. Dispositif selon l'une des revendications 3 à 12, avec un dispositif (22) de commande individuel des sources de rayonnement.

14. Dispositif selon l'une des revendications 3 à 13, avec des moyens de détermination des caractéristiques de la cornée.

15. Microscope pour opérations, relié à un dispositif selon l'une des revendications 3 à 14.

16. Appareil muni d'un système laser chirurgical destiné aux corrections réfractives de la cornée, en liaison avec un appareil selon l'une des revendications 1 à 15.
